# EUROPEAN PATENT APPLICATION

(11) **EP 4 778 584 A1**
(43) Date of publication of application: **22.07.2026**
(21) Application number: 25152750.3
(22) Date of filing: 20.01.2025
(51) Int. Cl.: A61P 31/02, A61K 8/20, A61C 17/00, A61C 19/00, A61K 33/20, A61Q 11/00

(54) **DISPOSABLE NON-CUSTOM ORAL TRAY USING HYPOCHLOROUS ACID GEL**

(71) Applicant: Koninklijke Philips N.V., 5656 AE Eindhoven (NL)
(72) Inventor: MAO, Jingliang, Eindhoven (NL)
(74) Representative: Philips Intellectual Property & Standards

(57) **Abstract**

An oral care system (200) comprising: a disposable tray (210) configured to deliver a hypochlorous acid gel (220) to at least a portion of teeth and/or gum soft tissue inside a user's oral cavity, the disposable tray comprising an interior surface having the hypochlorous acid gel thereon and configured to fit contours of at least the portion of the teeth and/or gum soft tissue of the user's oral cavity; wherein the hypochlorous acid gel comprises hypochlorous acid and a pH sensitive rheology modifying polymer, and wherein the hypochlorous acid gel comprises a pH of about 5.0 to about 6.5

## Description

### FIELD OF THE DISCLOSURE

The present disclosure is directed generally to an oral care system using a non-customized tray to deliver a hypochlorous acid gel treatment to a subject's oral cavity for oral hygiene care and treatment of gingivitis, periodontitis, peri-implant mucositis, and peri-implantitis.

### BACKGROUND

Gum care or periodontal health is an increasingly important healthcare topic. It encompasses healthy gingivae and periodontal attachment in the natural dentition and the health of their equivalent structures around dental implants. In oral health care, one of the most common types of dental diseases is gum disease including gingivitis, periodontitis, peri-implant mucositis and peri-implantitis. Nearly half of U.S. adults over the age of 30 show signs of gum disease, and nine percent have severe gum disease, known as periodontal disease. When left untreated, gum disease can become more difficult to remedy. Finding feasible solutions/products for gum care, especially gum diseases treatment has essential importance in oral health care.

Gingivitis, periodontitis, peri-implant mucositis, and peri-implantitis are bacterial inflammations with similar symptoms. The underlying cause is microbial plaque forming a biofilm which is rich in pathogenic bacteria. The National Institutes of Health (NIH) has reported that biofilms are responsible for up to 80% of human bacterial infection. Accordingly, removal of microbial plaque and calculus is essential to establish periodontal health, thus preventing periodontal disease and related tooth decay. Most importantly, the elimination of biofilm is vital for all treatment.

Current treatments for periodontal disease include brushing, mouthwash, flossing, antiseptics, antibiotics, or dental surgery. Use of antiseptic mouthwash including hydrogen peroxide, cetylpyridinium chloride (CPC), essential oils, or sodium hypochlorite is common practice. However, these treatments have significant limitations.

For example, there is limited effectiveness on an infected area, due to the recalcitrant nature of biofilm that has slime layers of high resistance from those pathogenic bacteria. To achieve the targeted disinfection and/or removal effect, it requires a long treatment time, a large volume of treatment liquid on gum/tooth surface, and/or avoiding eating or drinking anything for 30 minutes afterward.

Existing treatment methods also require higher minimum bactericidal concentration and longer killing time. For example, higher concentrations of hydrogen peroxide (10% to 30%) and longer contact times are required for sporicidal activity. Though sodium hypochlorite is much more effective than hydrogen peroxide in elimination of biofilm, research shows that even a solution of 1% of NaClO is not able to kill all the bacteria within five minutes, regardless of its killing temperature.

Existing treatment methods also have the risk of developing drug/antibiotic resistance. There is a high risk when an antibiotic product is frequently used for treatment of periodontal disease and peri-implant diseases, and when the antibiotic targets only specific bacterial molecules.

Existing treatment methods also see discomfort and inconvenience in use due to limited retention capability on the infected area, which is especially true for liquid type products. Long treatment times, large volumes of liquid inside the mouth or on gum/tooth surface, and/or avoiding eating or drinking anything afterward introduce discomfort and inconvenience to the patient. There can also be a bad or unpleasant taste or smell. For example, one of the main causes of unpopularity of sodium hypochlorite mouthwash is the bad taste. There is also the possibility of undesired side effects during use or from accidental swallowing. Existing treatment methods also have higher cost.

Accordingly, existing products and methods for treating periodontal disease are expensive, uncomfortable, and ineffective.

### SUMMARY OF THE DISCLOSURE

There is thus a continued need for improved compositions, methods, and systems to treat periodontal disease.

Various embodiments and implementations are directed to oral care systems and methods. The system and methods include a disposable tray to deliver a hypochlorous acid gel to the teeth and/or gum soft tissue inside a user's oral cavity. The disposable tray has an interior surface with hypochlorous acid gel, and is configured to fit contours of the user's dental arches. The hypochlorous acid gel comprises hypochlorous acid and a pH sensitive rheology modifying polymer, and has a pH of about 5.0 to about 6.5.

According to an aspect, an oral care system is provided. The system includes a disposable tray (210) configured to deliver a hypochlorous acid gel (220) to at least a portion of teeth and/or gum soft tissue inside a user's oral cavity, the disposable tray comprising an interior surface having the hypochlorous acid gel thereon and configured to fit contours of at least the portion of the teeth and/or soft tissue of the user's dental arches; wherein the hypochlorous acid gel comprises hypochlorous acid and a pH sensitive rheology modifying polymer, and wherein the hypochlorous acid gel comprises a pH of about 5.0 to about 6.5.

According to an embodiment, at least a portion of the disposable tray is flexible.

According to an embodiment, the disposable tray is not customized to contours of a specific user's oral cavity.

According to an embodiment, the hypochlorous acid gel further comprises a flavoring.

According to an embodiment, the hypochlorous acid gel is applied to the interior surface of the disposable tray just prior to delivering the hypochlorous acid gel to at least the portion of teeth and/or gum soft tissue.

According to an embodiment, the hypochlorous acid gel is formed prior to providing the disposable tray to the user.

According to an embodiment, the disposable tray is configured to fit the dental arc contours of a portion of a population.

According to another aspect is an oral care method. The method includes providing a disposable tray configured to deliver a hypochlorous acid gel to at least a portion of teeth and/or gum soft tissue inside a user's oral cavity, the disposable tray comprising an interior surface having the hypochlorous acid gel thereon and configured to fit contours of at least the portion of the teeth and/or gum soft tissue of the user's oral cavity, wherein the hypochlorous acid gel comprises hypochlorous acid and a pH sensitive rheology modifying polymer, and wherein the hypochlorous acid gel comprises a pH of about 5.0 to about 6.5; and applying the disposable tray to the at least the portion of teeth and/or gum soft tissue inside the user's oral cavity for a first period of time.

According to an embodiment, the method further comprises forming the hypochlorous acid gel; and applying the hypochlorous acid gel to the interior surface of the disposable tray.

According to an embodiment, forming the hypochlorous acid gel comprises mixing together a first component comprising: (i) sodium hypochlorite; and (ii) potassium hydroxide; and a second component comprising: (i) a pH sensitive rheology modifying polymer; and (ii) hydrochloric acid; wherein when the first component and the second component are combined into a mixture the hypochlorous acid gel is created with pH of about 5.0 to about 6.5.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
FIG. 1 is a flowchart of a method for oral care using hypochlorous acid gel, in accordance with an embodiment.
FIG. 2 is a schematic representation of an oral care tray, in accordance with an embodiment.
FIG. 3 is a schematic representation of an oral care tray, in accordance with an embodiment.
FIG. 4 is a schematic representation of an oral care tray, in accordance with an embodiment.
FIG. 5 is a schematic representation of an oral care tray, in accordance with an embodiment.
FIG. 6 is a schematic representation of an oral care tray, in accordance with an embodiment.

### DETAILED DESCRIPTION OF EMBODIMENTS

The present disclosure describes various embodiments of a composition for treating periodontal disease using a disposable tray configured to deliver a hypochlorous acid gel to the dental arches of a subject's oral cavity. More generally, Applicant has recognized and appreciated that it would be beneficial to provide an improved method and system for treating periodontal disease, leading to improved outcomes. The oral care systems and methods include a disposable tray to deliver a hypochlorous acid gel to the teeth and/or gum soft tissue inside a user's oral cavity. The disposable tray has an interior surface with hypochlorous acid gel, and is configured to fit the dental arch contours of the user's oral cavity. The hypochlorous acid gel comprises hypochlorous acid and a pH sensitive rheology modifying polymer, and has a pH of about 5.0 to about 6.

Hypochlorous acid (HOCl) is known to be significantly more effective and killing microorganisms much faster than hypochlorite ions (ClO⁻). In wound healing, for example, hypochlorous acid (HOCl) has been well known as an ideal wound care agent with powerful microbiocidal, antibiofilm, and wound healing potency. The minimum bactericidal concentration of stabilized HOCl tested at 37°C for 60 minutes is 3-6 ppm for most pathogenic microorganisms. At 218 ppm (0.0218%) of HOCl level, all microorganisms were killed within 12 seconds. In contrast, 1% of NaClO was not able to significantly kill bacteria within 5 minutes, regardless of its killing temperature. Moreover, hypochlorous acid is colorless, odorless, and tasteless, and it is classified as non-hazardous by the U.S. Environmental Protection Agency (EPA). It has also been confirmed in clinical studies to be non-toxic and non-irritating to the eye and skin. Accordingly, hypochlorous acid has great potential in oral health care.

The science behind the effectiveness of hypochlorous acid vs. hypochlorite is due to the difference in electrochemistry. Hypochlorous acid (HOCl) is electrically neutral while hypochlorite ions (ClO⁻) are electrically negative. This difference results in very distinct disinfection behaviors between them. For example, the cell wall of pathogenic microorganisms is negatively charged by nature. The negative charge of hypochlorite ions will be repulsed by the negative charge of the pathogenic microorganism cell wall, making it a weak disinfectant. Due to electrically neutrality, a hypochlorous acid molecule can very easily penetrate the cell wall of the pathogenic microorganism, thus making it a more effective disinfectant. In addition to its electrical neutrality, hypochlorous acid is also a much more reactive and stronger disinfectant than hypochlorite ions, as hypochlorous acid is split into hydrochloric acid (HCl) and oxygen, which in itself is a powerful disinfectant.

Hypochlorous acid (HOCl) can also easily penetrate slime layers, cell walls, and protective layers of microorganisms and thus effectively kills pathogens. The microorganisms will either die or suffer from reproductive failures.

The oral care systems and methods described or otherwise envisioned herein comprise a novel disposable product with a non-custom tray loaded with hypochlorous acid gel. It is a convenient, safe, cost-effective, and user-friendly oral care product for successful biofilm removal and elimination, resulting in better prevention and/or treatment of gum diseases and related tooth decay. In addition, since hypochlorous acid is a broad-spectrum disinfectant, antiseptic and deodorization agent, this method can be readily expanded to hypochlorous acid gel based sanitization and deodorization applications that require safer and shorter treatment times, which includes household, industrial, institutional, health care, and other fields.

The oral care systems and methods described or otherwise envisioned herein provide numerous advantages. The oral care systems and methods improve plaque biofilm elimination and removal. The oral care systems and methods are highly convenient and effective in prevention or treatment of gum diseases like periodontal disease & peri-implant diseases. The oral care systems and methods can also be modified for sanitization applications in other areas. The oral care systems and methods eliminate odor-producing bacteria that grow between gum and teeth and cause bad breath (halitosis), has a better taste, and has higher flexibility, more convenient, and lower cost.

There are several ways to apply oral care products, and specifically gum care products. According to one application method, the treatment is applied on the gum directly via spraying, painting, or rinsing approach with the product, e.g., rinsing with mouthwash. This is a very common practice for gum care products. Another application method is to use a customized tray made from a stone cast or via a boil-and-bite approach to apply the treatment. Yet another application method is to use active content-coated strips to apply the treatment. A fourth application method is to use non-customized tray to apply the gum care treatment, which is not seen often. Disposable gum care products using non-custom trays for periodontal disease treatment is rare.

According to an embodiment, the oral care product disclosed or otherwise envisioned herein is a disposable, pre-shaped, thin-walled, flexible, thermoplastic, non-custom dental tray preloaded with pre-formed hypochlorous acid gel or filled with an on-site-produced hypochlorous acid treatment gel just prior to placement over a subject's teeth and gum. The dental tray is thin, flexible, and non-custom, which allows it to be placed over a subject's entire teeth and covers approximately 50% to 99% area of gum, which is significantly different from existing disposable non-custom dental trays that generally don't cover the gum surface or if it covers, only a small areas of gum surface (i.e., less than ½ of gum surface above the gumline). The disposable pre-shaped gum care tray can be pre-loaded with a pre-formed hypochlorous acid gel and stored within a sealed packaging container. It can alternatively be filled with a hypochlorous acid treatment gel produced just prior to placement over a person's teeth and gum.

According to an embodiment, the oral care product performs like a customized tray but there is no need to go through a lengthy tray customization procedure. The materials for making a disposable non-custom tray can be one or more of ethylene-vinyl acetate copolymer (EVA), ethylene-vinyl alcohol copolymer (EVAL), polyvinyl chloride (PVC), polyethylene (PE) with different densities, polypropylene (PP), polycaprolactone (PCL), polyurethane, silicone, polystyrene, blend of waxes and polyolefins (like composition of Parafilm), polyesters, polycarbonates, polyamides, polyester amides, poly-tetrafluoroethylene, cellulose esters, polyvinyl acetate, polyvinyl alcohol, or other suitable thermoplastic polymer materials that are resistant to oxidizers at pH 4.5-8.0 range and are approved by FDA for food and/or medical applications. FDA approved plasticizers, flow additives, and fillers known in the art can be used as desired to modify the properties of the material used to form the disposable tray.

According to an embodiment, the shape and configuration of the oral tray can be a "Π," "Ω," "n," or "U" shape, preferably with both side walls' ends bent outward to cover the entire upper or lower dental arch surface (covering approximately up to 99% area of gum). The preferable two outward bent ends perform like two soft grips of the tray so the tray can gently create more clamp on the gums with the sticky gels and help hold the tray's gel contents against the teeth and gum surface more closely for disinfection. By design, on one hand, the shape and configuration of the tray makes more hypochlorous acid gel cover the interdental area, gingival sulcus, marginal gingiva, interdental gingiva and free gingiva where microbial plaque, biofilm, and pathogenic microorganisms mostly flourish and are harder to reach or be removed by brushing and flossing. On the other hand, the tray will have thinner or less hypochlorous acid gel that covers the surface of teeth and the attached gingiva areas where less microbial plaque, biofilm and pathogenic microorganisms attach to. Accordingly, the disinfection power/capacity of hypochlorous acid can focus on areas where it is most needed. The appearance of the trays can be colored, clear, opaque, or in-between.

With an acidity of pKa=7.53, hypochlorous acid is a weak acid that forms when chlorine dissolves in water and partially dissociates, starting to form hypochlorite ions (ClO⁻) when the pH goes above approximately 5.5. Hypochlorous acid cannot be isolated from solutions due to rapid equilibration with its precursor chlorine and its dissociated hypochlorite ions. There is a dynamic balance among Cl₂ molecules, HOCl molecules, and ClO⁻ anions in aqueous hypochlorous acid solution within pH 4 to 9. When an aqueous solution's pH is over 6.0, the proportion of hypochlorous acid declines from virtually 100% down to almost 0% at pH 9.0. Between pH 4.0 to 5.5, hypochlorous acid is at its maximum level. However, solutions with pH values lower than 5.0 will begin to generate and release potentially harmful levels of chlorine gas (Cl₂). Hypochlorous acid decreases when the pH is below 4 (to Cl₂ molecules) or above 5.5 (to ClO⁻ anions). Therefore, a pH between 5.0 and 6.0 is ideal for safe oral disinfection by hypochlorous acid, since an optimal level of hypochlorous acid (HCLO ≥ 95 %) is present and there is no chance for the generation and release of harmful chlorine gas within that pH range. Thus, a pH of 5.0-6.5 and especially pH 5.5-6.0 is desired for the pH of the hypochlorous acid gel. Furthermore, it is known that pH of human saliva ranges from 6.5 to 7.5. In application, even if the hypochlorous acid gel is eventually diluted by saliva in the treatment process, there is still a favorable oral environmental pH condition for over 50% of all hypochlorous acid molecules remaining active in the whole treatment process.

According to an embodiment, in order to create a hypochlorous acid-based gel, a thickening agent is necessary. Some pH sensitive polyacrylic acid derived thickeners are stable against low concentration ((≤ 10000 ppm (1%, wt./wt.) or ≤ 5000 ppm (0.5%, wt./wt.)) of hypochlorous acid and hypochlorite during long term storage at room temperature (59° - 77°F, or 15° - 25°C) and especially at refrigeration (34 - 40 °F or 1.1 - 4.4 °C) storage condition, while it can significantly thicken aquatic solution into highly viscous gel (>10,000 cps.) at pH 5.0 to pH 10.0, or pH 5.5 to pH 11.0 with appropriate dosage. It can instantly (5-10 seconds) or slowly (>10 seconds but <4 hours) thicken the desired formulation, depending on the thickener's dosage level and the speed of pH change of the formulation during compounding process. Specifically, before the gel formation/creation, that desired thickener exists as a stable, low viscosity ingredient. But when mixing that thickener with other component(s) of the system, the pH change (≥ pH 5.0 or ≥ pH 5.5) in the final mixture will trigger solution thickening due to electrostatic repulsion within thickener molecules, hydrophobic parts building associations with one another and with other hydrophobes available in the formulation, and/or crosslinking reaction. Instantly or slowly gelling around pH 5.0-6.0 to produce hypochlorous acid-based gel for the disposable gum care trays is well suited for the intended applications.

According to an embodiment, at least two types of pH sensitive, polyacrylic acid derived thickeners can be used. For example, some hydrophobically modified alkali soluble polymer emulsion (HASE) thickeners. HASE type polymer ACULYN^{™} 28 is stable in low concentration ((≤ 10000 ppm (wt./wt.), or ≤ 5000 ppm) of hypochlorite or hypochlorous acid. It thickens the formulation from pH 5.5 to pH 10. At pH around 5.8, it already reaches >95% of its thickening capacity. In product form, ACULYN^{™} 28 is supplied as low viscosity (<30 mPa•s) liquid emulsion with pH around 3.0. But it will rapidly thicken solution when the acid groups undergo ionization transitions from very acidic pH (pH< 5.0) to less acidic pH (pH 5.5 or above) or basic pH (7< pH <12). When their acid groups lose their protons and are neutralized, they become anionically charged and water-soluble, thus swelling/thickening due to dramatic charge-charge electrostatic repulsion. In addition, networking of numerous pendant hydrophobic groups of ACULYN^{™} 28 among themselves and with other hydrophobes in the formulation boosts their thickening effect. The final composition immediately thickens exponentially to become a high viscous gel. When the pH of the final composition is above 5.5, it remains a high viscosity gel.

Highly crosslinked polyacrylic acid polymers like Carbopol^{®} polymers are another possible thickening agent. Carbopol^{®} polymers, such as Carbopol^{®} 940 NF, thickens the formulation at pH 4.0 to pH 10. In that pH range, when carboxylic groups of Carbopol^{®} polymers lose their protons and are neutralized, they become anionically charged and water-soluble, thus swelling/thickening due to dramatic charge-charge electrostatic repulsion. Again, at pH 5.8, Carbopol^{®} 940 NF already reaches >95% of its thickening capacity.

In formulation, when an alkaline solution of low hypochlorite concentration (≤ 10000 ppm (wt./wt.), preferred ≤ 5000 ppm, with pH around 12.5) goes below pH 7.0 (around 5.0-6.5, pH 5.5-6.0 is preferred for application on human) after mixing with acidic solution containing the a thickener (like ACULYN^{™} 28 or Carbopol 940 NF) and pH modifying and buffering agent (such as citric acid, carbonic acid, acetic acid, phosphoric acid, or other compatible acid, or a base if needed), to final pH of around 5.8, it can thicken/turn the final mixed composition into a gel. The gelling speed is dependent on how the mixing process is carried out (instantly homogenously mixing or slowly mixing to change the pH of final mixture) and the speed of attaining targeted final pH of the finished product (gel).

Compared to existing dental gel methods and products, the methods and systems described or otherwise envisioned herein utilize hypochlorites, including low-concentration-hypochlorous-acid compatible and non-hazardous pH sensitive rheology modifying polymer(s) that thicken at pH 4.0 or above, and an acidic pH modifying agent to generate hypochlorous acid gels instantly or slowly via pH manipulation and mixing process control. It forms either pre-formed or instantly formed hypochlorous acid gel for disposable gum care applications.

Notably, each functional ingredient used in composition of this invention must be compatible with low concentration (≤1.0%) hypochlorous acid at room or refrigeration storage temperature (0°C < T < 5 °C. The final pH of product must be within pH 5.0-6.5, and a final pH 5.5-6.0 is desired for oral health applications.

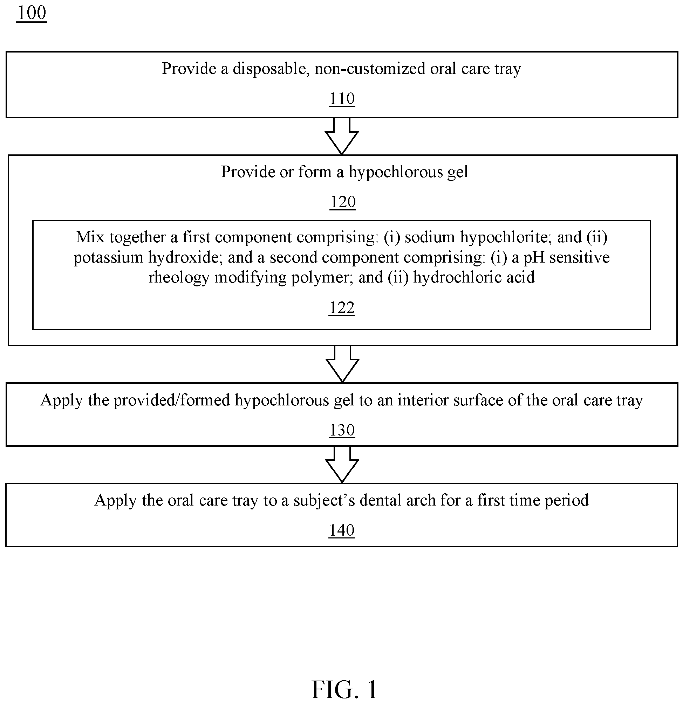

Referring to FIG. 1, in one embodiment, is a flowchart of a method 100 for treating or preventing periodontal disease using an oral care system. The methods described in connection with the figures are provided as examples only, and shall be understood not to limit the scope of the disclosure.

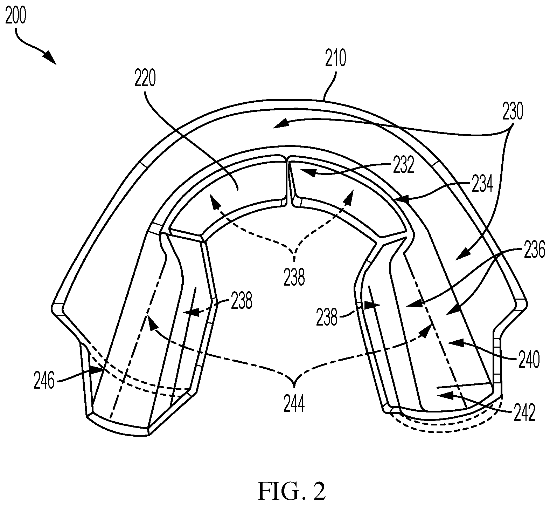

At step 110 of the method, an oral care system 200 is provided. Referring to an embodiment of an oral care system 200 as depicted in FIG. 2, for example, the system comprises an oral tray 210 and a hypochlorous acid gel 220. FIG. 2 depicts a simple schematic drawing of a perspective view of non-custom gum care tray for an upper dental arch. Referring to an embodiment of an oral care system 200 as depicted in FIG. 2, for example, the device or system comprises one or more of a labial or buccal wall 230, a notch/slit 232, a transition between posterior and anterior 234, an interior trough 236, a lingual wall(s) 238, a bottom wall 240, a closed end wall 242, an indentation 244, and/or a closed end wall 246. It will be understood that FIG. 2 constitutes, in some respects, an abstraction and that the actual organization of the components of the device or system 200 may be different and more complex than illustrated. Additionally, device or system 200 can be any of the devices or systems described or otherwise envisioned herein. Other elements and components of the device or system 200 are disclosed and/or envisioned elsewhere herein.

According to an embodiment, the oral tray 210 can be made from a wide variety of different material(s). For example, the tray can be made with thermoplastic polymers like ethylene-vinyl acetate copolymer (EVA), ethylene-vinyl alcohol copolymer (EVAL), polyvinyl chloride (PVC), polyethylene (PE) with different densities, polypropylene (PP), polycaprolactone (PCL), polyurethane, silicone, polystyrene, a blend of waxes and polyolefins, polyesters, polycarbonates, polyamides, polylactic acid (polylactide), polyester amides, poly-tetrafluoroethylene, cellulose esters, polyvinyl acetate, polyvinyl alcohol, and/or other suitable thermoplastic polymer materials that are able to resist low concentration oxidizers (≤ 10000 ppm or ≤ 1.0%) at pH 4.5 -8.0 and also approved by FDA for food and/or medical applications. Other FDA-approved plasticizers, flow additives, and fillers known in the art and compatible with low concentration (≤1.0%) hypochlorous acid can also be used as desired to modify the properties of the material used to form the tray. The tray can be colored, clear, opaque, or in-between in appearance. When the thickness of the tray wall is increased to a certain level, for example, ≥ 2mm, the oral care tray can even be made like a mouthguard device to apply the gum care gel on teeth and gum.

According to an embodiment, the oral tray 210 can comprise many different shapes and sizes. In summary, it can be made in a "U" shape, an "n" shape, or similar shapes in cross-sectional view and horseshoe-shaped in top view perspective, preferably with both ends bent outward to cover the entire upper or lower dental arch surface (covering approximately up to 99% area of gum of the upper/ maxillary arch or the lower/mandibular arch). The preferable two outward bent ends perform something like two soft grips of the tray with the sticky gel so the tray can have extra gently clamp to hold more onto the gums to help put tray's active gel contents against the teeth and gum surface more closely for disinfection. When the thickness of gum care tray wall is very thin (< 1.0 mm), using the exoskeleton can provide firm support to tray in application. The size, shape, appearance, and/or necessity of each component of the trays can be reduced, added, or modified to make it a fit and attractive device for commercial/applications purposes.

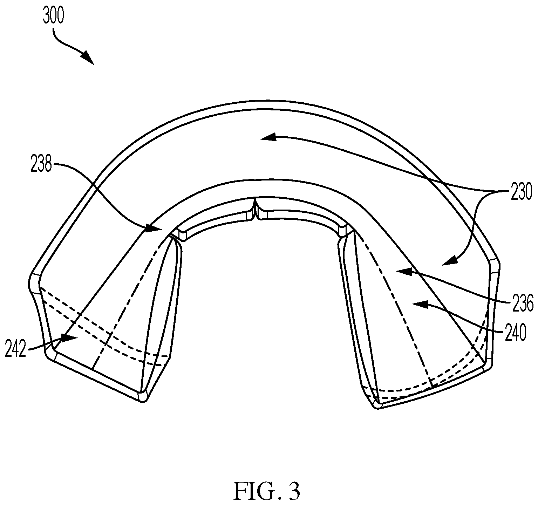

Referring to an embodiment of an oral care system 300 as depicted in FIG. 3, for example, the system comprises an oral tray and a hypochlorous acid gel (not shown, as it will be a gel applied to the interior of the tray). FIG. 3 depicts a simple schematic drawing of a perspective view of non-custom gum care tray for a lower dental arch. Referring to an embodiment of an oral care system 300 as depicted in FIG. 3, for example, the device or system comprises one or more of a labial or buccal wall 230, an interior trough 236, a lingual wall(s) 238, a bottom wall 240, and/or a closed end wall 242. It will be understood that FIG. 3 constitutes, in some respects, an abstraction and that the actual organization of the components of the device or system 300 may be different and more complex than illustrated.

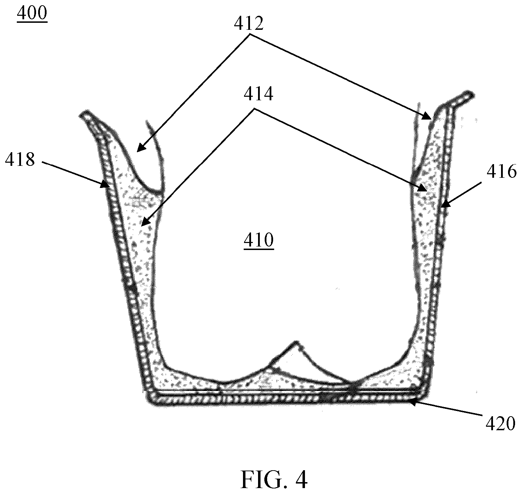

Referring to FIG. 4, in one embodiment, is a cross-sectional view of an oral care tray 400 filled with hypochlorous acid gel during treatment. Although not part of the oral care tray, FIG. 4 depicts a tooth 410 and gum 412 around which the tray is positioned. The oral care tray 400 comprises hypochlorous acid gel 414, a labial/buccal wall 416, a lingual wall 418, and a bottom wall 420.

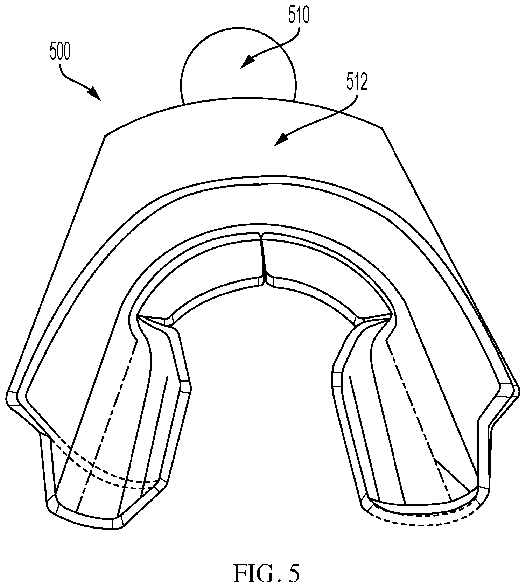

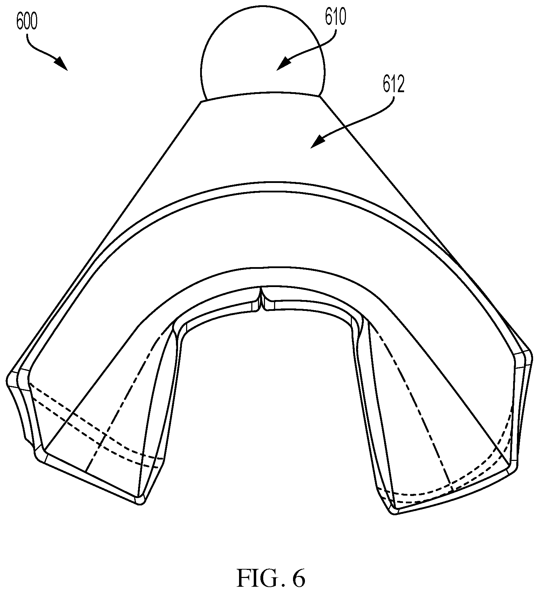

Referring to FIG. 5, in one embodiment, is a perspective view of an oral care tray 500 with an optional exoskeleton 512 and handle 510, for an upper dental arch. Similarly, referring to FIG. 6, in one embodiment, is a perspective view of an oral care tray 600 with an optional exoskeleton 612 and handle 610, for a lower dental arch.

According to an embodiment, the oral care tray (such as without an exoskeleton) can be pulled off the dental arch(es) by prying open via fingernail(s) or an appropriate firm tool after each treatment. Any residual hypochlorous acid gel on the gum & teeth can be removed by washing, flushing, and/or brushing.

According to an embodiment, the making of oral care tray can be accomplished via vacuum forming, pressing, or stamping from those thermoplastic polymer sheets and by but not limited to stone cast models, which are based on standard/universal models of upper & lower dental arches that are derived from a general population and can be made according to small, medium, and large size of dental arch of human.

### EXAMPLE

Provided herein is a general exemplary procedure to make a disposable, non-customized oral care tray as described or otherwise envisioned herein. Notably, this is only provided as an example and thus is non-limiting on the overall scope of the disclosure.

At step 1, make alginate/VPS impressions in small, medium, and large size based on models of upper & lower dental arches that are derived from a general population (for example, a population of a country (USA, Japan, China, France, ...), a gender (male or female), or any other delimiter).

At step 2, make stone casts or models of the impressions.

At step 3, vacuum form/press/stamp dental trays from the models, such as from a heated sheet of thin ethyl vinyl acetate (EVA) material or other suitable thermoplastic polymer sheets.

At step 4, form trays with 6-12 mm past gingival margin and trim any excess material. As shown in the drawings, if needed, make optional thin slit(s) or notch (es) (with slit/notch gap width ≤2.0 mm) at the lingual and/or bottom wall near the center of front teeth or at places where the bicuspids and canines meet. Those optional slit(s)/notch(es) can provide flexibility to make the non-custom tray more easily conform to dental arches of different sizes.

At step 5, wash in cool, soapy water and rinse with clean water, then cold sterilizing and packing those trays into sterile containers or filling those sterile trays with appropriate amount of pre-formed hypochlorous acid gel and packing them into sterile containers.

According to an embodiment, the methods described or otherwise envisioned herein result in a non-custom tray that is soft and flexible, fitting closely over the patient's teeth & gums, and that is therefore comfortable to wear for gum care. The resulting tray is quite economically feasible considering its non-custom and mass production features.

Returning to method 100 in FIG. 1, at step 120 of the method a hypochlorous acid gel is provided or formed. According to an embodiment where the hypochlorous acid gel is provided, such as providing a tube or container of hypochlorous acid gel that a clinician or user applies to the oral care tray, the gel is formed prior to the user's use of the oral care tray. For example, the gel can be prepared in advance by a manufacturer, and stored in a way that preserves the activity of the gel.

According to another embodiment, the hypochlorous acid gel is formed just prior to use by the user, and is thus immediately applied to the oral care tray just prior to application of the tray and the gel to at least a portion of teeth and/or soft tissue inside a user's oral cavity. Thus, at step 112 of the method, mix (122) a first component comprising: (i) sodium hypochlorite; and (ii) potassium hydroxide; with a second component comprising: (i) a pH sensitive rheology modifying polymer; and (ii) hydrochloric acid. According to an embodiment, when the first component and the second component are combined into a mixture, the hypochlorous acid gel is created with a pH of about 5.0 to about 6.5.

At step 130 of the method, when ready for application, the provided or formed hypochlorous acid gel is applied to the interior of the oral care tray. The hypochlorous acid gel can be pre-portioned such that an exact amount is known to be dispensed into the interior trough of the oral care tray, such as a first portion to be dispensed into an interior trough of an upper or lower portion of an oral care tray, and a second portion to be dispensed into an interior trough of the other section - i.e., the upper or lower portion - of the oral care tray.

At step 140 of the method, the oral care tray is positioned by a clinician or the user over at least a portion of teeth and/or soft tissue inside a user's oral cavity. For example, an upper portion of the oral care tray can be positioned such that it encompasses the user's upper teeth and at least a portion of the user's upper gums near the teeth. A lower portion of the oral care tray can be positioned such that it encompasses the user's lower teeth and at least a portion of the user's lower gums near the teeth. According to an embodiment, the oral care tray with the hypochlorous acid gel can remain on the user's teeth and gums for a predetermined amount of time, which may vary depending on a variety of factors including the specifics of the user, the concentration of the hypochlorous acid, and the periodontal disease being treated, among many other possible factors.

According to an embodiment, the hypochlorous acid gel can be made via several approaches. According to a first approach, the hypochlorous acid gel can be made on site, with an instantly formed hypochlorous acid gel resulting from the combination of two, three, four, or multi-parts and related dosing systems. According to a second approach, the hypochlorous acid gel can be pre-formed using a regular compounding process to fill a clean dispensing device such as syringes, bottles, vials, etc. TABLE 1 gives an example formulation example to make approximately 250 ppm hypochlorous acid gel with a pH approximately 5.8 using the second approach (i.e., making the hypochlorous acid gel on site).

**TABLE 1. Example hypochlorous acid gel formulation.**

| | Ingredient | % w/w in the whole formula |
|---|---|---|
| Part I | DI Water | 30.000% |
| | Sodium Hypochlorite 0.5% (wt.%) with 0.2% Excess NaOH | 7.095% |
| | Potassium Nitrate, USP | 2.000% |
| | 5.0 % Potassium Hydroxide, ACS or USP | 8.603% |
| Compounding Note: In Part I, add potassium nitrate into DI water solution of sodium hypochlorite, mix well until it is completely dissolved before slowly adding potassium hydroxide solution. That is the solution of Part I. | | |
| Part II | DI Water | 39.821% |
| | Potassium Nitrate, USP | 2.200% |
| | ACULYN^{™} 28 | 10.000% |
| | Nat. Peppermint Oil PE-5523 | 0.100% |
| | Hydrochloric Acid, 0.100% (wt.%) | 0.181% |
| Compounding Note: Pre-prepare the aqueous solution of all ingredients of Part II. Then, with strong mixing, add the solution of Part I into the solution of Part II. Continue mixing vigorously until a homogenous, viscous gel is produced. | | |

Please note that the above formulation example is just one simple formulation demonstration. The actual ingredients, formula, and compounding process can be minimized, added, or modified to make it a cost-effective solution for commercial purposes. Additionally, each functional/inert ingredient used in the composition must be compatible with low concentration (≤ 10000 ppm or ≤ 1.0%) of hypochlorous acid in long term (≥ 6 months) storage at room or refrigeration temperature. The final gel's pH (mixture of all ingredients in final gel form) must be within pH 5.0- 6.5, and a final gel pH within pH 5.5-6.0 is desired for oral health care applications.

According to an embodiment, one or both of the first component and the second component comprise a flavoring agent. Flavoring agents are substances that trigger the sense of taste and/or smell. They are used to improve the consumer perception and experience in treatment of dentine hypersensitivity. Generally, minty flavors such as peppermint oil and spearmint oil are used to give a sense of cleanliness and freshness for dental products. Sweeteners like xylitol or artificial sweeteners like sodium saccharin and sucralose are also used. A commonly preferred flavoring agents is peppermint oil. As long as a flavoring ingredient is compatible with other ingredients in the first component and/or the second component, it could be used at minimum dosage level if it is desired by applications and users.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An oral care system (200) comprising:
a disposable tray (210) configured to deliver a hypochlorous acid gel (220) to at least a portion of teeth and/or gum soft tissue inside a user's oral cavity, the disposable tray comprising an interior surface having the hypochlorous acid gel thereon and configured to fit contours of at least the portion of the teeth and/or gum soft tissue of the user's oral cavity;
wherein the hypochlorous acid gel comprises hypochlorous acid and a pH sensitive rheology modifying polymer, and wherein the hypochlorous acid gel comprises a pH of about 5.0 to about 6.5.

2. The oral care system of claim 1, wherein at least a portion of the disposable tray is flexible.

3. The oral care system of claim 1, wherein the disposable tray is not customized to contours of dental arches of a specific user's oral cavity.

4. The oral care system of claim 1, wherein the hypochlorous acid gel further comprises a flavoring.

5. The oral care system of claim 1, wherein the hypochlorous acid gel is applied to the interior surface of the disposable tray just prior to delivering the hypochlorous acid gel to at least the portion of teeth and/or gum soft tissue.

6. The oral care system of claim 1, wherein the hypochlorous acid gel is formed prior to providing the disposable tray to the user.

7. The oral care system of claim 1, wherein the disposable tray is configured to fit contours of dental arches of a portion of a population.

8. An oral care method (100), comprising:
providing (110) a disposable tray (200) configured to deliver a hypochlorous acid gel to at least a portion of teeth and/or gum soft tissue inside a user's oral cavity, the disposable tray comprising an interior surface having the hypochlorous acid gel thereon and configured to fit contours of at least the portion of the teeth and/or gum soft tissue of the user's oral cavity, wherein the hypochlorous acid gel comprises hypochlorous acid and a pH sensitive rheology modifying polymer, and wherein the hypochlorous acid gel comprises a pH of about 5.0 to about 6.5; and
applying (140) the disposable tray to the at least the portion of teeth and/or gum soft tissue inside the user's oral cavity for a first period of time.

9. The oral care method of claim 8, further comprising the steps of:
forming (120) the hypochlorous acid gel; and
applying (130) the hypochlorous acid gel to the interior surface of the disposable tray.

10. The oral care method of claim 9, wherein forming the hypochlorous acid gel comprises:
mixing (122) together a first component comprising: (i) sodium hypochlorite; and (ii) potassium hydroxide; and a second component comprising: (i) a pH sensitive rheology modifying polymer; and (ii) hydrochloric acid;
wherein when the first component and the second component are combined into a mixture the hypochlorous acid gel is created with pH of about 5.0 to about 6.5.

11. The oral care method of claim 8, wherein at least a portion of the disposable tray is flexible.

12. The oral care method of claim 8, wherein the disposable tray is not customized to contours of dental arches of a specific user's oral cavity.

13. The oral care method of claim 8, wherein the hypochlorous acid gel further comprises a flavoring.

14. The oral care method of claim 8, wherein the hypochlorous acid gel is applied to the interior surface of the disposable tray just prior to delivering the hypochlorous acid gel to at least the portion of teeth and/or gum soft tissue.

15. The oral care method of claim 8, wherein the hypochlorous acid gel is formed prior to providing the disposable tray to the user.
